# EUROPEAN PATENT APPLICATION

(11) **EP 4 620 487 A2**
(43) Date of publication of application: **24.09.2025**
(21) Application number: 25164930.7
(22) Date of filing: 20.03.2025
(51) Int. Cl.: A61L 2/18

(54) **REPROCESSING DEVICE AND METHOD FOR REPROCESSING A MEDICAL INSTRUMENT**

(30) Priority: 21.03.2024 DE 102024108120
(71) Applicant: Olympus Winter & Ibe GmbH, 22045 Hamburg (DE)
(72) Inventor: Oebbeke, Dirk, 32547 Bad Oeynhausen (DE); Wolff, Marcel, 22111 Hamburg (DE); Karabacak, Ibrahim, 22041 Hamburg (DE); Hopf, Oliver, 25421 Pinneberg (DE)
(74) Representative: Eisenführ Speiser

(57) **Abstract**

The invention relates to a reprocessing device for reprocessing a medical instrument and to a method for reprocessing a medical instrument and to a use of a reprocessing device. The reprocessing device (1) comprises a reprocessing chamber (10) for receiving a medical instrument. The reprocessing chamber comprises an inlet port (12) and an outlet port (11). A circulation line system (20) comprises a first connecting line end (21) and a second connecting line end (22). The first connecting line end is connected to the outlet port (11) of the reprocessing chamber and the second connecting line end is connected to the inlet port (12) of the reprocessing chamber. A circulation device (30) is connected to a circulation line (25) of the circulation line system and adapted to circulate a flushing fluid through the circulation line system from the outlet port (11) of the reprocessing chamber to the inlet port (12) of the reprocessing chamber. A sampling device (40) for removing a sample of flushing fluid from the circulation line system (20) is connected to a circulation line (25) of the circulation line system. The sampling device comprises a valve arrangement (41) that is connected to the circulation line system (20) for removing flushing fluid from the circulation line system.

## Description

The invention relates to a reprocessing device for reprocessing a medical instrument. Further, the invention relates to a method for reprocessing a medical instrument and to a use of a reprocessing device.

After use of a medical instrument such as an endoscope, the used and contaminated endoscope is typically reprocessed by pre-cleaning, automated cleaning, disinfection, and possibly sterilization, before it can be used again on the patient. In order to minimize or avoid risks of insufficient cleaning and/or insufficient disinfection, reprocessing devices are used. Reprocessing devices are used to reprocess medical instruments, such as endoscopes. Reprocessing devices are in particular used to conduct an automated cleaning and disinfection of medical instruments, in particular endoscopes. Such reprocessing devices for endoscopes can also be called endoscope reprocessors or automated endoscope reprocessors. Reprocessing devices allow for an automated reliable cleaning and high-level disinfection of medical instruments such as endoscopes. With such a reprocessing device, foreign matter can effectively be removed from the medical instrument.

However, to maintain reprocessing performance of the reprocessing device, the disinfectant's minimum recommended concentration (MRC) of a disinfectant solution in the flushing fluid must be checked on a regular basis, in particular prior to each reprocessing cycle. Typically test strips are used to test whether the concentration of the disinfectant solution in the flushing fluid used has at least the MRC to ensure that the disinfectant solution is effective for disinfection. Typically, via a disinfectant solution nozzle that is located below the lid of the reprocessing device, disinfectant solution can be supplied to a reprocessing basin of the reprocessing device. To check whether the disinfectant concentration meets the recommended level the disinfectant solution nozzle can be opened to insert a test strip. The test strips are then evaluated visually, in particular the test strips are compared to a defined color scale to determine the concentration of disinfectant solution.

A problem of known reprocessing devices is that checking the MRC is only possible if the reprocessing device is completely stopped, i.e. when no reprocessing takes place. For checking the MRC, the lid of the reprocessing device has to be opened and a lid of the disinfectant solution nozzle has to be removed. Therefore, checking the MRC is relatively time-consuming and labor-intensive. Furthermore, the user conducting the check can come in direct contact with the relatively aggressive disinfectant solution which poses a risk to the user. In addition, it is possible that the user contaminates a medical instrument that has already been reprocessed and that is still arranged inside the reprocessing device. A further problem is the possibility that the user could forget to close the disinfectant solution nozzle after cleaning which could result in an unwanted outflow.

Therefore, it is an object of the present invention to provide an improved reprocessing device for reprocessing a medical instrument and an improved method for reprocessing a medical instrument. In particular, it is an object of the present invention to provide a solution with which checking the concentration of disinfectant solution can be conducted in a more flexible way while minimizing risks posed to a user and risks due to incorrect operation.

According to a first aspect, it is provided a reprocessing device for reprocessing a medical instrument, preferably a surgical medical instrument, in particular an endoscope, the reprocessing device comprising: a reprocessing chamber for receiving a medical instrument, preferably a surgical medical instrument, in particular an endoscope, wherein the reprocessing chamber comprises an inlet port and an outlet port, a circulation line system that comprises a first connecting line end and a second connecting line end, wherein the first connecting line end is connected to the outlet port of the reprocessing chamber and the second connecting line end is connected to the inlet port of the reprocessing chamber, a circulation device, preferably a circulation pump, that is connected to a circulation line of the circulation line system and adapted to circulate a flushing fluid through the circulation line system from the outlet port of the reprocessing chamber to the inlet port of the reprocessing chamber, a sampling device for removing a sample of flushing fluid from the circulation line system, wherein the sampling device is connected to a circulation line of the circulation line system, wherein the sampling device comprises a valve arrangement that is connected to the circulation line system for removing flushing fluid from the circulation line system.

The reprocessing device is preferably configured to reprocess a medical instrument, preferably a surgical medical instrument, in particular an endoscope.

The reprocessing chamber is preferably adapted to receive a medical instrument, preferably a surgical medical instrument, in particular an endoscope. The reprocessing chamber is preferably formed to receive and hold a medical instrument, preferably a surgical medical instrument, in particular an endoscope. The reprocessing chamber has an inlet port and an outlet port. Preferably, the reprocessing chamber is formed as a reprocessing basin.

It is particularly preferred that the reprocessing device comprises a disinfectant solution tank for storing disinfectant solution, wherein the disinfectant solution tank is fluidly connected and/or connectable to the reprocessing chamber. Via such a disinfectant solution tank, disinfectant solution can be provided and introduced into the reprocessing chamber.

The circulation line system is connected to the outlet port of the reprocessing chamber and to the inlet port of the reprocessing chamber. It is possible that further inlet ports are provided. For example, it is possible that the reprocessing chamber comprises two inlet ports that are arranged at different positions of the reprocessing chamber. The circulation line system can comprise one or more circulation lines.

The circulation device is preferably arranged and adapted to circulate a flushing fluid through the circulation line system from the outlet port of the reprocessing chamber to the inlet port of the reprocessing chamber. The flushing fluid is in particular to be understood to comprise the disinfectant solution.

The sampling device is preferably arranged and adapted to allow for removing a sample of flushing fluid from the circulation line system. The valve arrangement of the sampling device is preferably connected to the circulation line system in such a way that flushing fluid can be removed from the circulation line system. The sampling device is preferably connected to the circulation line of the circulation line system to which the circulation device is connected. Thus, the sampling device and the circulation device are preferably connected to the same circulation line of the circulation line system.

An advantage of this solution is that the sampling device allows to access the flushing fluid, and thus to remove flushing fluid and test the flushing fluid, in particular by using test strips. With the sampling device, it is possible to access the flushing fluid without the need to open the lid of the reprocessing device and thus without the need to completely stop the reprocessing device. It is therefore possible to analyze and/or test the flushing fluid during a reprocessing procedure. Thus, the productivity can be increased and testing can be conducted more flexible in time.

Another advantage of this solution is that the sampling device provides an access to the flushing fluid with which hygiene risks as well as safety risks for the user can be significantly reduced when compared to existing solutions.

Furthermore, in particular compared to automated measuring systems, e.g. comprising measuring sensors for checking the MRC, that are relatively complex and expensive, the suggested solution is relatively inexpensive and at the same time very reliable. Furthermore, the system can be designed relatively robust resulting in relatively low maintenance costs.

It is preferred that the sampling device is connected to the circulation line system in proximity to the circulation device, and preferably behind the circulation device in relation to a flow direction of flushing fluid that extends through the circulation line system from the outlet port to the inlet port.

Preferably, the circulation device is configured to move the flushing fluid in one flow direction, namely from the outlet port through the circulation line system to the inlet port. Preferably, the sampling device is arranged behind the circulation device with respect to the flow direction of the flushing fluid.

An advantage of arranging the sampling device behind the circulation device is that this way the circulation device can be stopped so that the user can open the sampling device and remove flushing fluid, in particular by inserting a test strip into the flushing fluid that is present in the circulation line system. Then, the sampling device can be closed and the circulation device can be started again to move the flushing fluid again in the flow direction.

It is preferred that the circulation line to which the valve arrangement of the sampling device is connected to comprises a circulation line opening. The circulation line opening can be formed as a bore and/or a hole, in particular a circular hole. Such a circulation line opening allows for access to the flushing fluid and thus to test the flushing fluid.

Preferably, the valve arrangement comprises a check valve that is arranged and adapted to close the circulation line opening and to prevent flushing fluid to flow out of the circulation line opening. Preferably, the check valve is a one-way valve. Preferably, the check valve is a spring loaded check valve. An advantage of using such a check valve is that this way it can reliably be ensured that no flushing fluid exits the circulation line opening unless the check valve is opened.

It is preferred that the valve arrangement comprises a valve member, wherein the valve member is movable between an opened valve position, in which the valve member opens the circulation line opening, and a closed valve position, in which the valve member closes the circulation line opening, wherein preferably the valve arrangement comprises a valve seat and the valve member is in direct contact with the valve seat when arranged in the closed valve position and not in direct contact with the valve seat when arranged in the opened valve position.

Preferably, in the opened valve position, the valve member opens the circulation line opening in such a way that flushing fluid can be removed from the circulation line system, in particular by inserting a test strip through the circulation line opening and then removing a test strip. Preferably, in the closed valve position, the valve member closes the circulation line opening so that no flushing fluid can be removed from the circulation line system, so that in particular inserting a test strip is not possible.

The valve member can in particular adjustably restrict fluid flow through the valve arrangement. Preferably the valve member can move linearly inside the valve arrangement or rotate on a stem, or rotate on a hinge ortrunnion. The valve member is preferably formed as a disc or as a ball.

The valve seat the seat is preferably part of the circulation line opening, i.e. formed by the circulation line opening. The valve seat is preferably the interior surface of the body which contacts the valve member to form a leak-tight seal. If the valve member moves linearly or swings on a hinge or trunnion, the valve member comes into contact with the valve seat only when the valve is closed.

It is preferred that the valve member is biased to be moved towards the circulation line opening so that the valve member is biased to be moved in the closed valve position, preferably by using a spring element, preferably a compression spring, in particular a coil spring, wherein the spring element applies a closing force to the valve member so that the valve member is moved in the closed valve position.

Preferably, if the valve member is not actuated, the valve member is arranged in the closed valve position. Preferably, the spring element is a compression spring formed as a coil spring that applies a compression force as closing force to the valve member so that the valve member is moved in the closed valve position. With such a biased valve member, in particular a reliable closure of the circulation line opening can be achieved.

It is preferred that the valve arrangement comprises a pull rod, wherein the pull rod is connected to the valve member for manually moving the valve member from the closed valve position into the opened valve position against the closing force, wherein preferably the pull rod extends from a first end to a second end, wherein the first end of the pull rod is connected to the valve member and the second end of the pull rod is connected to a handle bar.

Preferably, the pull rod and the handle are arranged orthogonal to each other, in particular as to form a T-shaped arrangement. This way, the handling of the valve arrangement for a user can be improved.

It is preferred that the circulation line opening is connected to a pipe socket, wherein the valve arrangement is connected to the pipe socket, wherein the pipe socket has a pipe socket opening for inserting a test strip through the pipe socket opening and through the circulation line opening when the valve member is arranged in the opened valve position, wherein the pipe socket opening is fluidly connected to the circulation line opening when the valve member is arranged in the opened valve position.

Preferably, the pipe socket opening is arranged as an opening on an outer circumferential wall of the pipe socket. Preferably, the pipe socket extends orthogonal to the extension of the circulation line in which the circulation line opening is arranged.

It is preferred that the valve arrangement comprises a check valve that is formed as a duckbill valve, wherein preferably the duckbill valve has at least two converging flaps, that are preferably flexible and preferably comprise or consist of rubber and/or elastomer, in particular the at least two converging flaps are flexible in such a way that a test strip can be moved between the at least two converging flaps and through an opening of the at least two converging flaps that is formed by the test strip when moved between the at least two converging flaps.

Preferably, a duckbill valve is understood to be a check valve, preferably manufactured from rubber or synthetic elastomer. Preferably, the duckbill valve that has two or more converging flaps that are shaped like the beak of a duck. The duckbill valve can be formed as a joker valve.

Preferably, the duckbill valve is arranged and adapted to close the circulation line opening and to prevent flushing fluid to flow out of the circulation line opening. Preferably, a test strip can be inserted through the duckbill valve, and then in particular further through the circulation line opening.

An advantage of using such a duckbill valve is that it can be avoided that when the valve arrangement is opened fluid escapes or air is sucked in, depending on the pressure conditions. As both of these situations are undesirable, a duckbill valve can be particularly preferably. With such a duckbill valve, hygienic safety can be guaranteed in a particularly preferred way as a formation of bio-films can be prevented.

It is preferred that the reprocessing device comprises a bypass line that is connected at a first connecting point to the circulation line of the circulation line system and connected at a second connecting point to the circulation line of the circulation line system, wherein at least one valve is connected to the bypass line, wherein the at least one valve is arranged and adapted to fluidly connect and fluidly disconnect the bypass line to the circulation line of the circulation line system.

An advantage of providing such a bypass line is that the bypass line can be fluidly connected and disconnected from the circulation line of the circulation line system by using the at least one valve. This way, when disconnecting the bypass line from the circulation line, flushing fluid can be removed from the bypass line, independently of any fluid flow that is present in the circulation line of the circulation line system.

The bypass line preferably serves to separate the fluid from the circulation line of the circulation line system to ensure targeted sampling. By using such a bypass line, it can be ensured that only the solution from a disinfection step is withdrawn and tested. This can in particular be achieved by opening the at least one valve after a disinfection step has been conducted and closing the at least one valve again after a predefined time.

It is preferred that the at least one valve is a time-controlled valve. Preferably, the at least one valve is opened and closed time-controlled, preferably dependent on the reprocessing cycle.

It is preferred that the bypass line has a bypass opening, wherein a capillary tube is connected to the bypass opening, wherein the capillary tube is arranged and adapted to remove fluid from the bypass line by using the capillary effect, in particular for providing fluid to be tested by using a test strip.

Preferably, the capillary tube is used to remove flushing fluid from the bypass line by using the capillary effect. Preferably, the capillary tube is integrated in the bypass line and/or connected to the bypass line. Preferably, after flushing fluid has been removed using the capillary tube, the removed sample is, in particular automatically, poured on a test strip. Therefore, the test strip can in particular be held in an ideal position, preferably by a test strip holding device.

It is preferred that the capillary tube extends from the bypass line to a container that is formed as a leakage container or connected to a leakage container.

Preferably, while pouring a test solution of removed flushing fluid on a test strip, residual test solution is collected by using the leakage container and then discharged.

Preferably the leakage container is fluidly connected to a drainage system. Preferably, the leakage container can be removed and/or discharged manually and/or the leakage container can be connected to a drainage system to discharge the leakage container.

It is preferred that the reprocessing device is designed as a top-loader, the reprocessing device comprising a lid that is arranged on top of the reprocessing device, wherein the lid is movable, preferably by rotation about a horizontal axis, between an open position and a closed position, wherein preferably the lid extends in a substantially horizontally plane when being arranged in the closed position.

Preferably the valve arrangement is arranged at the front side of the reprocessing device.

The reprocessing device preferably has a top side, on which the lid of the reprocessing device is arranged, and further a front side, two side walls, and a back side.

Arranging the valve arrangement at the front side of the reprocessing device allows for an easy access to the valve arrangement by a user so that conducting a test by using a test strip is particularly easy and fast conducted. Furthermore, such an arrangement is beneficial, because the lid can be left in the closed position during testing.

According to a further aspect it is provided a method for reprocessing a medical instrument, preferably a surgical medical instrument, in particular an endoscope, wherein the method comprises the following steps: Providing a reprocessing device, preferably a reprocessing device as described herein, wherein the reprocessing device comprises: a reprocessing chamber for receiving a medical instrument, preferably a surgical medical instrument, in particular an endoscope, wherein the reprocessing chamber comprises an inlet port and an outlet port, a circulation line system, a circulation device, preferably a circulation pump, that is connected to a circulation line of the circulation line system and adapted to circulate a flushing fluid through the circulation line system, and a sampling device for removing a sample of flushing fluid from the circulation line system, wherein the sampling device is connected to a circulation line of the circulation line system, wherein the sampling device comprises a valve arrangement that is connected to the circulation line system for removing flushing fluid from the circulation line system; Extracting a sample liquid from the flushing fluid through an opening of the circulation line to which the sampling device is connected; Applying the extracted sample liquid onto a test strip; Analyzing the test strip to determine if the concentration of disinfectant solution in the extracted sample liquid lies above or below a predetermined concentration threshold.

Preferably, the method for reprocessing a medical instrument is conducted as part of a method for using a medical instrument, wherein the method for using a medical instrument comprises the steps of: Moving at least a part of the surgical instrument, in particular of an endoscope, into a body, preferably a human body, and conducting a surgical step within the body by using the medical instrument; Moving the surgical instrument out of the body; and then Conducting a method for reprocessing the medical instrument as described herein.

Preferably the flushing fluid is extracted during a disinfection step. This is preferably achieved by opening at least one valve after a disinfection step has been conducted and closing the at least one valve again after a predefined time, i.e. by controlling the at least one valve in a time-controlled manner.

Preferably the step of Analyzing the test strip comprises optically comparing the color of the test strip with a color scale.

It is preferred that Extracting a sample liquid through the opening of the circulation line to which the sampling device is connected comprises: Moving a valve member of the valve arrangement from a closed valve position, in which the valve member closes the circulation line opening, to an opened valve position, in which the valve member opens the circulation line opening, and preferably Holding the valve member in the opened valve position against a closing force that is applied to the valve member to move the valve member into the closed valve position.

Preferably, the valve member is biased into the closed valve position, preferably by using a spring element.

It is preferred that Applying the extracted sample liquid onto the test strip comprises: Inserting the test strip at least partly through the circulation line opening and into the flushing liquid, preferably by Inserting the test strip through a pipe socket opening of a pipe socket that is connected to the valve arrangement and through the circulation line opening in such a way that the test strip is partly arranged inside the circulation line.

It is preferred that Moving the valve member of the valve arrangement from the closed valve position to the opened valve position is conducted after stopping the circulation device that leads to a stop of circulation of the flushing fluid through the circulation line system.

It is preferred that the valve arrangement comprises a check valve that is formed as a duckbill valve, wherein preferably the duckbill valve has at least two converging flaps, that are preferably flexible and preferably comprise or consist of rubber and/or elastomer, wherein test strip is moved between the at least two converging flaps so that it extends through the duckbill valve and through the circulation line opening and extends partly into the inside of the circulation line.

It is preferred that a part of the flushing liquid is moved into a bypass line that is connected to the circulation line of the circulation line system, wherein at least one valve is connected to the bypass line, wherein preferably the at least one valve opens the valve in a time-controlled manner.

Preferably the sample liquid is removed from the bypass line by using a capillary tube that is connected to a bypass opening of the bypass line, wherein the sample liquid being removed from the bypass line is then applied onto the test strip.

According to a further aspect it is provided a use of a reprocessing device as described herein for cleaning a medical instrument, preferably a surgical medical instrument, in particular an endoscope, particularly preferred a flexible endoscope, wherein a sample liquid is extracted from flushing fluid in a circulation line system, and wherein test strips are used to determine if the concentration of disinfectant solution in the extracted sample liquid lies above or below a predetermined concentration threshold.

Preferably, dependent on the result, a currently conducted reprocessing can be continued or stopped.

As to the advantages, preferred embodiments and details of the individual different aspects and their preferred embodiments, reference is also made to the corresponding advantages, preferred embodiments and details described with reference to the respective other aspects.

Further advantageous embodiments result from the combination of individual, several or all of the preferred features described herein. Preferred embodiments shall now be described with reference to the attached drawings, in which
- Fig. 1:: shows a schematic view of a reprocessing device;
- Fig. 2a:: shows three detail views of a sampling device with a valve member, wherein the valve member is in a closed valve position;
- Fig. 2b:: shows three detail views of the sampling device shown in Fig. 2a, wherein the valve member is in an opened valve position;
- Fig. 2c:: shows a side view of the sampling device shown in Fig. 2a and Fig. 2b and a test strip;
- Fig. 3:: shows an embodiment of a check valve that is formed as a duckbill valve;
- Fig. 4:: shows an embodiment of a part of the reprocessing device comprising a bypass line;
- Fig. 5:: shows an embodiment of a part of the reprocessing device comprising a capillary tube;
- Fig. 6:: shows a schematic perspective view of a reprocessing device;
- Fig. 7:: shows a flow chart of a method for reprocessing a medical instrument;
- Fig. 8:: shows a flow chart of a method for reprocessing a medical instrument.

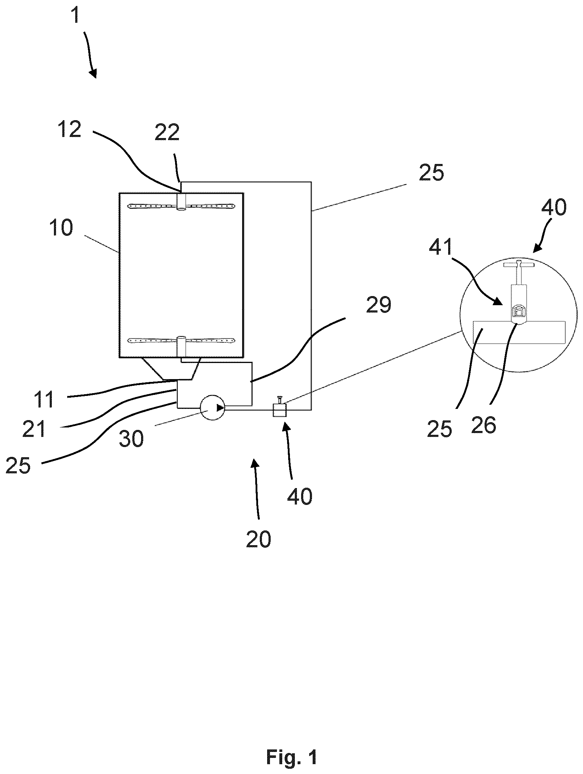

Fig. 1 shows a schematic view of a reprocessing device 1. The reprocessing device 1 is adapted to reprocess an endoscope. The reprocessing device 1 comprises a reprocessing chamber 10 for receiving an endoscope (not shown). The reprocessing chamber 10 comprises an inlet port 12 and an outlet port 11. A circulation line system 20 comprises a first connecting line end 21 and a second connecting line end 22. The first connecting line end 21 is connected to the outlet port 11 of the reprocessing chamber 10 and the second connecting line end 22 is connected to the inlet port 12 of the reprocessing chamber 10. A circulation device 30 that is a circulation pump is connected to a circulation line 25 of the circulation line system 20. The circulation device 30 is adapted to circulate a flushing fluid through the circulation line system 20 from the outlet port 11 of the reprocessing chamber 10 to the inlet port 12 of the reprocessing chamber 10. Furthermore, a backflow line 29 is provided that is connected to the circulation device 30 and to the reprocessing chamber 10. A sampling device 40 is provided for removing a sample of flushing fluid from the circulation line system 20. The sampling device 40 is connected to the circulation line 25. The sampling device 40 comprises a valve arrangement 41 that is connected to the circulation line system 20 for removing flushing fluid from the circulation line system 20. The circulation line 25 to which the valve arrangement 41 of the sampling device 40 is connected comprises a circulation line opening 26. Arrow F shows the flow direction of flushing fluid.

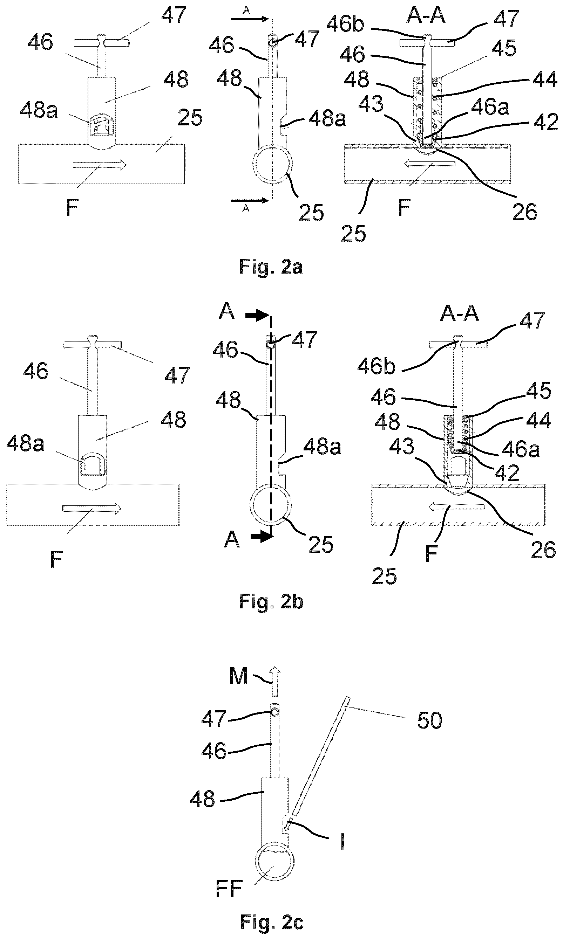

Fig. 2a shows three detail views of a sampling device shown in Fig. 2a with a valve member, wherein the valve member is in a closed valve position. The sampling device has a valve arrangement 41 that comprises a valve member 42, wherein the valve member 42 is movable between an opened valve position, in which the valve member 42 opens the circulation line opening 26, and a closed valve position, in which the valve member 42 closes the circulation line opening 26. The valve arrangement 41 comprises a valve seat 43 and the valve member 42 is in direct contact with the valve seat 43 when arranged in the closed valve position, which is the position shown in Fig. 2a. In the closed valve position, the valve member 42 closes the circulation line opening 26 so that no flushing fluid can be removed from the circulation line system, so that inserting a test strip is not possible.

In Fig. 2b, the valve arrangement 41 is shown in the opened valve position, wherein the valve member 42 opens the circulation line opening 26 in such a way that flushing fluid can be removed from the circulation line system by inserting a test strip through the circulation line opening 26 and then removing a test strip. The valve member 42 is biased to be moved towards the circulation line opening 26 so that the valve member 42 is biased to be moved in the closed valve position by using a spring element 44 that is formed as a compression spring designed as a coil spring. The valve arrangement comprises a pull rod 46. The pull rod 46 is connected to the valve member 42 at its first end 46a for manually moving the valve member 42 from the closed valve position into the opened valve position against the closing force. The second end 46b of the pull rod 46 is connected to a handle bar 47. The circulation line opening 26 is connected to a pipe socket 48. The valve arrangement is connected to the pipe socket 48, wherein the pipe socket has a pipe socket opening 48a for inserting a test strip through the pipe socket opening and through the circulation line opening when the valve member is arranged in the opened valve position. The pipe socket 48 is closed by a closure element 45 through which the pull rod 46 extends.

Fig. 2c shows a side view of the sampling device as described in the context of Fig. 2a and 2b. In Fig. 2c, the valve arrangement 41 is shown in the opened valve position. Furthermore, a test strip 50 is shown that can be inserted into the pipe socket opening 48a and further through the circulation line opening along direction I.

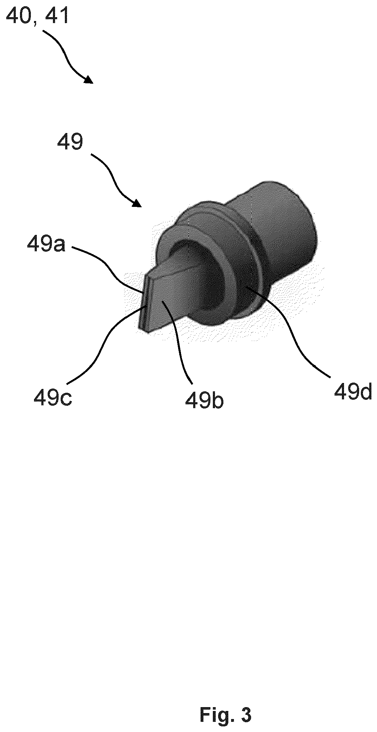

Fig. 3 shows an embodiment of a check valve that is formed as a duckbill valve. The valve arrangement 41 can comprise or be formed as a check valve 49 that is formed as a duckbill valve. The duckbill valve 49 has two converging flaps 49a, 49b, that are flexible and comprise or consist of rubber and/or elastomer. The two converging flaps 49a, 49b are flexible in such a way that a test strip can be moved between the at least two converging flaps 49a, 49b and through an opening 49c of the at least two converging flaps 49a, 49b. The opening is formed by a test strip when moved between the at least two converging flaps 49a, 49b by elastically moving the two converging flaps 49a and 49b. The duckbill valve 49 has a circular attachment body 49d for securing the duckbill valve 49 to the circulation line opening.

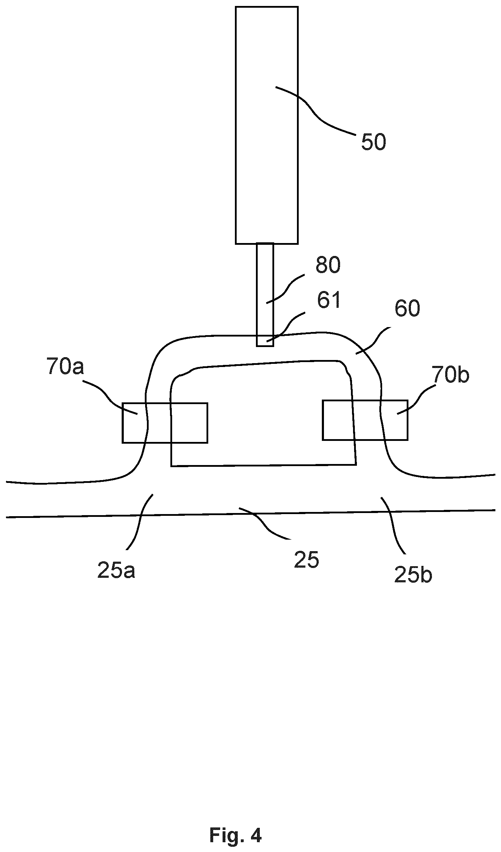

Fig. 4 shows an embodiment of a part of the reprocessing device comprising a bypass line 60 that is connected at a first connecting point 25a to the circulation line 25 of the circulation line system and connected at a second connecting point 25b to the circulation line 25 of the circulation line system. The valves 70a, 70b are connected to the bypass line 60, wherein the valves 70a, 70b are arranged and adapted to fluidly connect and fluidly disconnect the bypass line 60 to the circulation line 25 of the circulation line system. The valves 70a, 70b can be time-controlled valves. The bypass line 60 has a bypass opening 61, wherein a capillary tube 80 is connected to the bypass opening 61. The capillary tube 80 is arranged and adapted to remove fluid from the bypass line 60 by using the capillary effect. The removed fluid TF to be tested can then be tested by using a test strip 50.

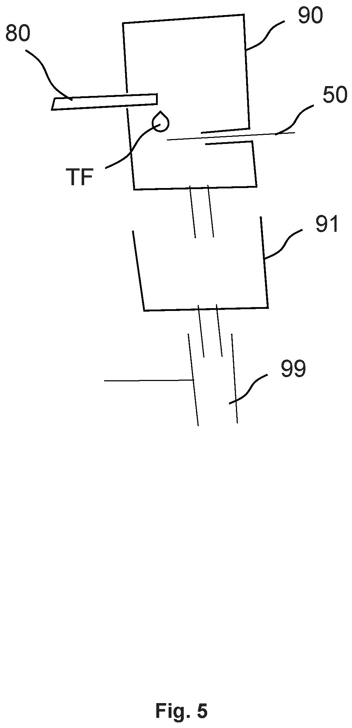

Fig. 5 shows an embodiment of a part of the reprocessing device comprising a capillary tube 80. The capillary tube 80 extends from the bypass line to a container 90 that is connected to a leakage container 91. The leakage container 91 is fluidly connected to a drainage system 99 to discharge the leakage container 91.

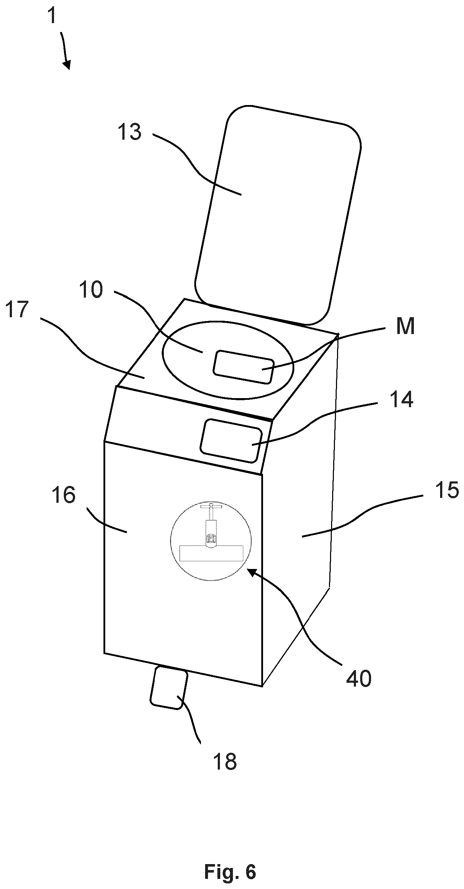

Fig. 6 shows a schematic perspective view of a reprocessing device 1. The reprocessing device 1 is designed as a top-loader. The reprocessing device 1 comprises a lid 13 that is arranged on top of the reprocessing device 1. The lid 13 is movable by rotation about a horizontal axis between an open position and a closed position. A foot pedal 18 is provided to open and/or close the lid 13. In Fig. 6 the lid 13 is shown in the open position. The reprocessing device 1 has a top side 17, on which the lid 13 of the reprocessing device 1 is arranged. Furthermore, the reprocessing device 1 has a front side 16, side walls 15, and a back side. A medical instrument M is arranged in the reprocessing chamber 10 that is formed as a reprocessing basin. A display 14 is provided to display information to a user. The sampling device 40 is arranged at the front side 16 of the reprocessing device 1 and therefore accessible from the front side 16 of the reprocessing device 1.

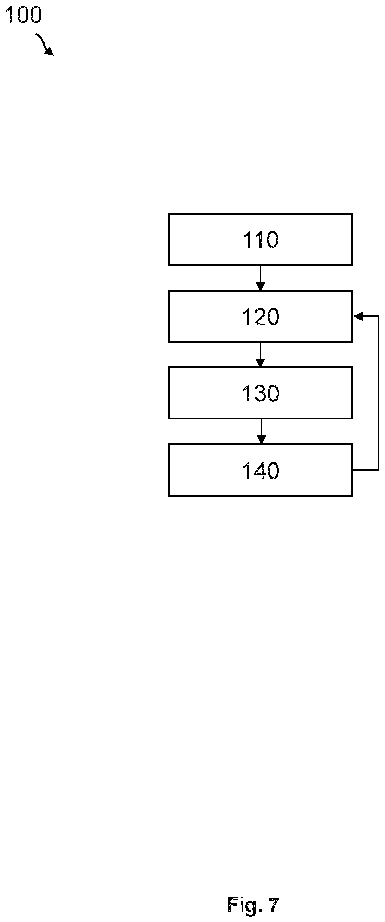

Fig. 7 shows a flow chart of a method 100 for reprocessing a medical instrument. The method 100 comprises the following steps:
In a step 110, Providing a reprocessing device, preferably a reprocessing device as described herein, wherein the reprocessing device comprises: a reprocessing chamber for receiving a medical instrument, preferably a surgical medical instrument, in particular an endoscope, wherein the reprocessing chamber comprises an inlet port and an outlet port, a circulation line system, a circulation device, preferably a circulation pump, that is connected to a circulation line of the circulation line system and adapted to circulate a flushing fluid through the circulation line system, and a sampling device for removing a sample of flushing fluid from the circulation line system, wherein the sampling device is connected to a circulation line of the circulation line system, wherein the sampling device comprises a valve arrangement that is connected to the circulation line system for removing flushing fluid from the circulation line system.

In a step 120, Extracting a sample liquid from the flushing fluid through an opening of the circulation line to which the sampling device is connected.

In a step 130, Applying the extracted sample liquid onto a test strip.

In a step 140, Analyzing the test strip to determine if the concentration of disinfectant solution in the extracted sample liquid lies above or below a predetermined concentration threshold.

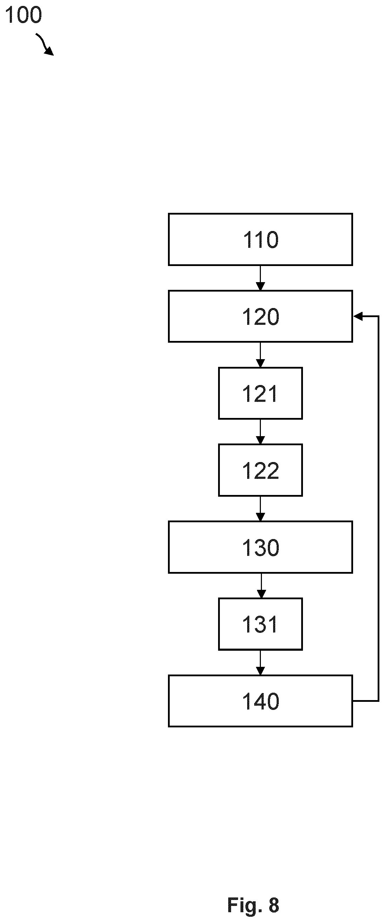

Fig. 8 shows a flow chart of a method 100 for reprocessing a medical instrument. The method 100 comprises the steps described in the context of Fig. 7. However, between the steps 120 and 130, the following steps 121 and 122 are included:
In a step 121, Moving a valve member of the valve arrangement from a closed valve position, in which the valve member closes the circulation line opening, to an opened valve position, in which the valve member opens the circulation line opening.

In a step 122, Holding the valve member in the opened valve position against a closing force that is applied to the valve member to move the valve member into the closed valve position.

Furthermore, between the steps 130 and 140, the step 131 is included:
In a step 131, Inserting the test strip at least partly through the circulation line opening and into the flushing liquid, preferably by Inserting the test strip through a pipe socket opening of a pipe socket that is connected to the valve arrangement and through the circulation line opening in such a way that the test strip is partly arranged inside the circulation line.

### List of Reference Signs

- 1: reprocessing device
- 10: reprocessing chamber
- 11: outlet port of reprocessing chamber
- 12: inlet port of reprocessing chamber
- 13: lid of reprocessing device
- 14: display of reprocessing device
- 15: side walls of reprocessing device
- 16: front side of reprocessing device
- 17: top side of reprocessing device
- 18: foot pedal of reprocessing device
- 20: circulation line system
- 21: first connecting line end
- 22: second connecting line end
- 25: circulation line of circulation line system
- 25a: first connecting point
- 25b: second connecting point
- 26: circulation line opening
- 29: backflow line
- 30: circulation device
- 40: sampling device
- 41: valve arrangement
- 42: valve member
- 43: valve seat
- 44: spring element
- 45: closure element
- 46: pull rod
- 46a: first end of pull rod
- 46b: second end of pull rod
- 47: handle bar
- 48: pipe socket
- 48a: pipe socket opening
- 49: check valve
- 49a, 49b: converging flaps of check valve
- 49c: opening of converging flaps of check valve
- 49d: circular attachment body of check valve
- 50: test strip
- 60: bypass line
- 61: bypass opening
- 70a, 70b: valves
- 80: capillary tube
- 90: container
- 91: leakage container
- 99: drainage system
- 100: method for reprocessing a medical instrument
- 110-140: method steps
- F: flow direction of flushing fluid
- I: direction for inserting a test strip
- M: medical instrument
- TF: removed flushing fluid

## Claims

1. A reprocessing device (1) for reprocessing a medical instrument, preferably a surgical medical instrument, in particular an endoscope, the reprocessing device comprising:
- a reprocessing chamber (10) for receiving a medical instrument, preferably a surgical medical instrument, in particular an endoscope, wherein the reprocessing chamber comprises an inlet port (12) and an outlet port (11),
- a circulation line system (20) that comprises a first connecting line end (21) and a second connecting line end (22), wherein the first connecting line end is connected to the outlet port (11) of the reprocessing chamber and the second connecting line end is connected to the inlet port (12) of the reprocessing chamber,
- a circulation device (30), preferably a circulation pump, that is connected to a circulation line (25) of the circulation line system and adapted to circulate a flushing fluid through the circulation line system from the outlet port (11) of the reprocessing chamber to the inlet port (12) of the reprocessing chamber,
- a sampling device (40) for removing a sample of flushing fluid from the circulation line system (20), wherein the sampling device is connected to a circulation line (25) of the circulation line system, wherein the sampling device comprises a valve arrangement (41) that is connected to the circulation line system (20) for removing flushing fluid from the circulation line system.

2. Reprocessing device according to at least one of the preceding claims,
wherein the sampling device (40) is connected to the circulation line system (20) in proximity to the circulation device (30), and preferably behind the circulation device in relation to a flow direction of flushing fluid that extends through the circulation line system from the outlet port (11) to the inlet port (12).

3. Reprocessing device according to at least one of the preceding claims,
wherein the circulation line (25) to which the valve arrangement (41) of the sampling device (40) is connected to comprises a circulation line opening (26),
wherein preferably the valve arrangement (41) comprises a check valve that is arranged and adapted to close the circulation line opening (26) and to prevent flushing fluid to flow out of the circulation line opening (26),
wherein preferably the valve arrangement (41) comprises a valve member (42), wherein the valve member is movable between an opened valve position, in which the valve member (42) opens the circulation line opening (26), and a closed valve position, in which the valve member (42) closes the circulation line opening (26), wherein preferably the valve arrangement (41) comprises a valve seat (43) and the valve member (42) is in direct contact with the valve seat when arranged in the closed valve position and not in direct contact with the valve seat when arranged in the opened valve position.

4. Reprocessing device according to claim 3,
wherein the valve member (42) is biased to be moved towards the circulation line opening (26) so that the valve member is biased to be moved in the closed valve position, preferably by using a spring element (44), preferably a compression spring, in particular a coil spring, wherein the spring element applies a closing force to the valve member so that the valve member is moved in the closed valve position, and/or
wherein the valve arrangement comprises a pull rod (46), wherein the pull rod is connected to the valve member for manually moving the valve member from the closed valve position into the opened valve position against the closing force, wherein preferably the pull rod extends from a first end (46a) to a second end (46b), wherein the first end of the pull rod is connected to the valve member (42) and the second end of the pull rod is connected to a handle bar (47).

5. Reprocessing device according to at least one of the preceding claims, wherein the circulation line opening (26) is connected to a pipe socket (48), wherein the valve arrangement (41) is connected to the pipe socket (48), wherein the pipe socket has a pipe socket opening (48a) for inserting a test strip (50) through the pipe socket opening and through the circulation line opening when the valve member is arranged in the opened valve position, wherein the pipe socket opening is fluidly connected to the circulation line opening when the valve member is arranged in the opened valve position.

6. Reprocessing device according to at least one of the preceding claims, wherein the valve arrangement comprises a check valve (49) that is formed as a duckbill valve, wherein preferably the duckbill valve has at least two converging flaps (49a, 49b), that are preferably flexible and preferably comprise or consist of rubber and/or elastomer, in particular the at least two converging flaps are flexible in such a way that a test strip can be moved between the at least two converging flaps and through an opening (49c) of the at least two converging flaps that is formed by the test strip when moved between the at least two converging flaps,
wherein preferably, the duckbill valve is arranged and adapted to close the circulation line opening (26) and to prevent flushing fluid to flow out of the circulation line opening.

7. Reprocessing device according to at least one of the preceding claims, wherein the reprocessing device comprises a bypass line (60) that is connected at a first connecting point (25a) to the circulation line (25) of the circulation line system (20) and connected at a second connecting point (25b) to the circulation line (25) of the circulation line system (20), wherein at least one valve (70a, 70b) is connected to the bypass line (60), wherein the at least one valve is arranged and adapted to fluidly connect and fluidly disconnect the bypass line to the circulation line of the circulation line system.

8. Reprocessing device according to at least one of the preceding claims, wherein the at least one valve (70a, 70b) is a time-controlled valve, and/or wherein the bypass line has a bypass opening (61), wherein a capillary tube (80) is connected to the bypass opening (61), wherein the capillary tube is arranged and adapted to remove fluid from the bypass line by using the capillary effect, in particular for providing fluid to be tested by using a test strip,
wherein preferably the capillary tube (80) extends from the bypass line (60) to a container (90) that is formed as a leakage container or connected to a leakage container (91),
wherein preferably the leakage container is fluidly connected to a drainage system (99).

9. **Reprocessing** device according to one of the preceding claims, wherein the reprocessing device is designed as a top-loader, the reprocessing device comprising a lid (13) that is arranged on top of the reprocessing device, wherein the lid is movable, preferably by rotation about a horizontal axis, between an open position and a closed position, wherein preferably the lid extends in a substantially horizontally plane when being arranged in the closed position,
wherein preferably the valve arrangement is arranged at the front side (16) of the reprocessing device.

10. A method (100) for reprocessing a medical instrument, preferably a surgical medical instrument, in particular an endoscope, wherein the method comprises the following steps:
- Providing (110) a reprocessing device, preferably a reprocessing device according to at least one of the preceding claims, wherein the reprocessing device comprises:
∘ a reprocessing chamber for receiving a medical instrument, preferably a surgical medical instrument, in particular an endoscope, wherein the reprocessing chamber comprises an inlet port and an outlet port,
∘ a circulation line system,
∘ a circulation device, preferably a circulation pump, that is connected to a circulation line of the circulation line system and adapted to circulate a flushing fluid through the circulation line system, and
∘ a sampling device for removing a sample of flushing fluid from the circulation line system, wherein the sampling device is connected to a circulation line of the circulation line system, wherein the sampling device comprises a valve arrangement that is connected to the circulation line system for removing flushing fluid from the circulation line system;
- Extracting (120) a sample liquid from the flushing fluid through an opening of the circulation line to which the sampling device is connected;
- Applying (130) the extracted sample liquid onto a test strip;
- Analyzing (140) the test strip to determine if the concentration of disinfectant solution in the extracted sample liquid lies above or below a predetermined concentration threshold.

11. Method according to the preceding claim, wherein Extracting (120) a sample liquid through the opening of the circulation line to which the sampling device is connected comprises:
o Moving (121) a valve member of the valve arrangement from a closed valve position, in which the valve member closes the circulation line opening, to an opened valve position, in which the valve member opens the circulation line opening, and preferably Holding (122) the valve member in the opened valve position against a closing force that is applied to the valve member to move the valve member into the closed valve position.

12. Method according to at least one of the preceding claims, wherein Applying (130) the extracted sample liquid onto the test strip comprises:
o Inserting (131) the test strip at least partly through the circulation line opening and into the flushing liquid, preferably by Inserting the test strip through a pipe socket opening of a pipe socket that is connected to the valve arrangement and through the circulation line opening in such a way that the test strip is partly arranged inside the circulation line.

13. Method according to at least one of the preceding claims, wherein Moving (121) the valve member of the valve arrangement from the closed valve position to the opened valve position is conducted after stopping the circulation device that leads to a stop of circulation of the flushing fluid through the circulation line system, and/or
wherein the valve arrangement comprises a check valve that is formed as a duckbill valve, wherein preferably the duckbill valve has at least two converging flaps, that are preferably flexible and preferably comprise or consist of rubber and/or elastomer, wherein test strip is moved between the at least two converging flaps so that it extends through the duckbill valve and through the circulation line opening and extends partly into the inside of the circulation line.

14. Method according to at least one of the preceding claims, wherein a part of the flushing liquid is moved into a bypass line that is connected to the circulation line of the circulation line system, wherein at least one valve is connected to the bypass line, wherein preferably the at least one valve opens the valve in a time-controlled manner,
wherein preferably the sample liquid is removed from the bypass line by using a capillary tube that is connected to a bypass opening of the bypass line, wherein the sample liquid being removed from the bypass line is then applied onto the test strip.

15. Use of a reprocessing device according to at least one of the claims 1-9 for cleaning a medical instrument, preferably a surgical medical instrument, in particular an endoscope, particularly preferred a flexible endoscope, wherein a sample liquid is extracted from flushing fluid in a circulation line system, and wherein test strips are used to determine if the concentration of disinfectant solution in the extracted sample liquid lies above or below a predetermined concentration threshold.
